# EUROPÄISCHE PATENTSCHRIFT

(11) **EP 3 510 323 B1**
(45) Veröffentlichungstag und Bekanntmachung des Hinweises auf die Patenterteilung: **23.11.2022**
(21) Anmeldenummer: 17768372.9
(22) Anmeldetag: 29.08.2017
(51) Int. Cl.: F21V 21/28, F21V 21/40, F21V 23/04, A61B 90/30, H05B 45/20, F21W 131/202, F21W 131/205, A61B 90/35

(54) **OPERATIONSLEUCHTE MIT MITTELN ZUR ABSTANDSMESSUNG**
SURGERY LIGHT WITH MEANS FOR DISTANCE MEASUREMENT
DISPOSITIF D'ÉCLAIRAGE D'UN CHAMP OPÉRATOIRE AVEC DES MOYENS DE MESURE DE DISTANCE

(30) Priorität: 12.09.2016 DE 102016117067
(43) Veröffentlichungstag der Anmeldung: 17.07.2019
(73) Patentinhaber: Karl Leibinger Medizintechnik GmbH & Co. KG, 78570 Mühlheim (DE)
(72) Erfinder: STRÖLIN, Joachim, 78570 Mühlheim (DE)
(74) Vertreter: Winter, Brandl - Partnerschaft mbB
(86) Internationale Anmeldenummer: PCT/EP2017/071593
(87) Internationale Veröffentlichungsnummer: WO 2018/046339

(56) Entgegenhaltungen:
- EP-A2- 0 422 331
- EP-A2- 0 422 331
- DE-A1- 10 225 077
- DE-A1- 10 225 077
- DE-U1- 20 316 755
- DE-U1- 20 316 755
- US-A1- 2012 161 647
- US-A1- 2012 161 647
- US-A1- 2015 145 419

## Beschreibung

Die Erfindung betrifft eine Operationsleuchte nach dem Oberbegriff des Anspruchs 1 mit einer Leuchteneinheit, die eine Vielzahl einzelner in einem Gehäuse aufgenommener Lichtquellen aufweist, wobei jede Lichtquelle so angeordnet und ausgerichtet ist, dass sie in einem bestromten Zustand einen (vorzugsweise zu einer gemeinsamen gedachten horizontalen Beleuchtungsebene hin gerichteten) Lichtstrahl erzeugt, mit einem Verankerungselement, das zum Befestigen an einer Decke, einer Wand oder einem Boden eines Raumes vorbereitet ist, sowie mit einem die Leuchteneinheit verschiebbar und/oder verschwenkbar an dem Verankerungselement anbindenden Haltearmsystem.

Aus dem Stand der Technik sind bereits gattungsgemäße Operationsleuchten prinzipiell bekannt. Beispielsweise offenbart die US 6 880 957 B2 ein Beleuchtungsgerät mit einer elektronischen Schattenkompensation. Weiterer relevanter Stand der Technik ist aus der EP 0 422 331 A2, der DE 102 25 077 A1, der US 2012/161647 A1, der DE 203 16 755 U1 sowie der US 2015/145419 A1 bekannt.

Eine Voraussetzung für eine optimale Beleuchtungseinstellung der Operationsleuchte ist, dass der Abstand zwischen der Leuchteneinheit der Operationsleuchte und dem Operationsfeld, das etwa durch eine Auflagefläche eines Operationstisches gebildet ist, bekannt sein sollte. Kommt es zu einer Veränderung der Position der Leuchteneinheit relativ zu dem Operationsfeld während einer Operation, muss die Beleuchtungseinstellung häufig möglichst rasch nachgestellt / nachgeführt werden.

Es gab daher bereits Überlegungen, Abstandssensoren an Operationsleuchten anzubringen, die den direkten Abstand zwischen der entsprechenden Leuchteneinheit und dem Operationsfeld ermitteln. Diese Sensoren messen intervallartig den Abstand zwischen der Operationsleuchte und dem Operationsfeld und ermöglichen dadurch ein rasches Nachstellen der Beleuchtungseinstellung. Die direkte Messung des Abstandes zwischen der Operationsleuchte und dem Operationsfeld / dem Lichtfeld ist jedoch relativ problematisch, da in den Messfelderfassungsbereich der verwendeten Abstandssensoren, zum Operationsfeld hin, häufig Störobjekte, wie Köpfe oder Hände der Operateure, während des Betriebes der Operationsleuchte eintreten. Dies führt häufig zu einer Fehlmessung sowie zu einem unerwünschten Nachstellen der Beleuchtungseinstellung der Operationsleuchte.

Es ist daher die Aufgabe der vorliegenden Erfindung, diese aus dem Stand der Technik bekannten Nachteile zu beheben und insbesondere eine Operationsleuchte zur Verfügung zu stellen, die ihre Beleuchtungseinstellung bei einer Lageveränderung der Leuchteneinheit möglichst rasch und genau nachstellt.

Dies wird erfindungsgemäß durch den kennzeichnenden Teil des Anspruchs 1 gelöst. Demnach ist eine Lagedetektionseinrichtung derart angeordnet sowie beschaffen, dass sie im Betriebszustand einen Abstand eines Referenzabschnittes der Leuchteneinheit oder des Haltearmsystems hin zu der Decke hin ermittelt. Die Lagedetektionseinrichtung weist einen ersten Abstandserfassungssensor auf, wodurch der Aufbau der Operationsleuchte besonders einfach gehalten ist. Die Raumdecke bildet unmittelbar eine große Fläche aus, die besonders einfach durch den Abstandserfassungssensor erfasst wird. Der erste Abstandserfassungssensor ist somit, in der Einbaulage der Operationsleuchte betrachtet, an einem im Raum nach oben gerichteten (in der Lage / Höhe verstellbaren) Abschnitt des Haltearmsystems oder der Leuchteneinheit angeordnet. Ferner weist das Gehäuse (der Leuchteneinheit) eine gekrümmt / gebogen verlaufende Erstreckung auf, wobei die durch die Lichtquellen erzeugten Lichtstrahlen zu einer gemeinsamen Lichtaustrittsseite des Gehäuses hin (aus der Leuchteneinheit) austreten. Die Lichtaustrittsseite ist auch als Unterseite der Leuchteneinheit bezeichnet. Dadurch ist die Lagedetektionseinrichtung besonders effektiv wirksam. Des Weiteren ist der erste Abstandserfassungssensor auf einer der Lichtaustrittsseite abgewandten Seite, d.h. einer Oberseite des Gehäuses angebracht (und weiter bevorzugt mit seinem Messfelderfassungsbereich entgegen den Lichtstrahlen der Lichtquellen ausgerichtet / von den Lichtstrahlen der Lichtquellen weg gerichtet ist). Dadurch ist der Aufbau der Operationsleuchte möglichst einfach gehalten, wobei die Lagedetektionseinrichtung geschickt in der Leuchteneinheit integriert ist.

Dies ermöglicht eine weitestgehend störungsfreie Referenzmessung zur Ermittlung eines Abstandes relativ zu einer Raumdecke. Durch diese Ausbildung ist die Operationsleuchte weniger anfällig für vor dem Operationsfeld hineinragende Gegenstände und es lässt sich ein weitestgehend störungsfreier Betrieb der Operationsleuchte umsetzen.

Weitere vorteilhafte Ausführungsformen sind in den Unteransprüchen beansprucht und nachfolgend näher erläutert.

Wenn der erste Abstandserfassungssensor im Bereich einer (höhenverstellbaren) Schwenkachse, um die die Leuchteneinheit relativ zu dem Haltearmsystem schwenkbar gelagert ist, angeordnet ist, ist der erste Abstandserfassungssensor an einer Stelle der Leuchteneinheit angebracht, die möglichst selten zur Raumdecke hin von Objekten verdeckt wird.

Weist die Lagedetektionseinrichtung, zusätzlich oder alternativ zu dem ersten Abstandserfassungssensor, einen zweiten Abstandserfassungssensor auf, der an dem Haltearmsystem vorgesehen ist, wird die Verlässlichkeit zur Ermittlung der Lage der Leuchteneinheit weiter verbessert.

In diesem Zusammenhang ist es wiederum zweckmäßig, wenn der zweite Abstandserfassungssensor an einem (höhenverstellbaren) Kardangelenk des Haltearmsystems angeordnet ist. In diesem Bereich kann die Abstandsmessung besonders präzise durchgeführt werden, sodass die Erfassungsanfälligkeit weiter gesenkt wird.

Auch ist es von Vorteil, wenn die Lagedetektionseinrichtung, zusätzlich oder alternativ zu dem ersten und/oder zweiten Abstandserfassungssensor einen (vorzugweise in dem Haltearmsystem angeordneten) Winkelerfassungssensor aufweist, der vorzugsweise so beschaffen und angeordnet ist, dass er einen Winkel / eine Winkellage eines verschwenkbar zu dem Verankerungselement angeordneten Tragarmabschnittes des Haltearmsystems (relativ zu einer Referenzgeraden / -achse) erfasst. Durch eine Rückbeziehung auf den entsprechenden Winkel des Tragarmabschnittes, der vorzugsweise ein höhenverstellbarer Federarm des Haltearmsystems ist, wird die Abstandsmessung weiter vereinfacht.

Vorteilhaft ist es, wenn die Lagedetektionseinrichtung mit einer in dem Gehäuse der Leuchteneinheit angebrachten / aufgenommenen Steuereinheit datenübermittelnd, vorzugsweise elektrisch, verbunden ist. Auf diese Weise wird die Lagedetektionseinrichtung nahe an der Steuereinheit angeordnet, sodass der Herstellaufwand der Operationsleuchte weiter gesenkt wird.

Wenn die Steuereinheit wiederum so steuernd mit den Lichtquellen gekoppelt ist, dass (im Betriebszustand) zumindest eine Lichteigenschaft der Leuchteneinheit, wie eine erzeugte Lichtfeldgröße, eine Lichtfarbe oder eine Anzahl der bestromten Lichtquellen, in Abhängigkeit eines durch die Steuereinheit ausgegebenen Steuerbefehls eingestellt / einstellbar ist. Dadurch werden die entsprechenden Lichteigenschaften der Leuchteneinheit besonders direkt angesteuert.

Ist die Steuereinheit zudem so programmiert, dass sie die zumindest eine Lichteigenschaft in Abhängigkeit des mittels der Lagedetektionseinrichtung in einem Betriebszustand der Operationsleuchte ermittelten Abstandes zwischen dem Referenzabschnitt und der Raumdecke einstellt, wird die Ansteuerung weiter vereinfacht.

Die Steuereinheit ist zudem vorteilhafterweise mit einer Rücksetzungseinrichtung verbunden, die es während des Betriebs der Operationsleuchte ermöglicht, einen Soll-Zustand der Operationsleuchte zu definieren, indem ein Ist-Zustand des Abstands relativ zu der Raumdecke erfasst wird und gleichzeitig die zumindest eine / mehrere eingestellte Lichteigenschaft/-en der Leuchteneinheit abgespeichert wird / werden. Die Rücksetzungseinrichtung weist weiter bevorzugt einen Rücksetzknopf oder eine Sprachbefehlseingabeeinrichtung, wie einen Lautsprecher, auf und ist wiederum mit der Steuereinheit elektrisch / datenübermittelnd verbunden. Dadurch wird die Ansteuerung der Operationsleuchte weiter vereinfacht.

In anderen Worten ausgedrückt, wird im Betrieb einer erfindungsgemäßen Operationsleuchte die Leuchteneinheit der Operationsleuchte zunächst optimal relativ zu einem Operationsfeld eingestellt und bspw. ein Referenzpunkt gesetzt, d.h. eine Referenzmessung zur Raumdecke durchgeführt. Verändert sich beim Umfunktionieren der Operationsleuchte der Abstand relativ zu der Raumdecke, werden die Beleuchtungseinstellungen / Lichteigenschaften entsprechend nachgeführt und angepasst / optimiert.

Die Erfindung wird nun nachfolgend anhand einer Figur näher erläutert, in welchem Zusammenhang auch verschiedene Ausführungsbeispiele beschrieben sind.

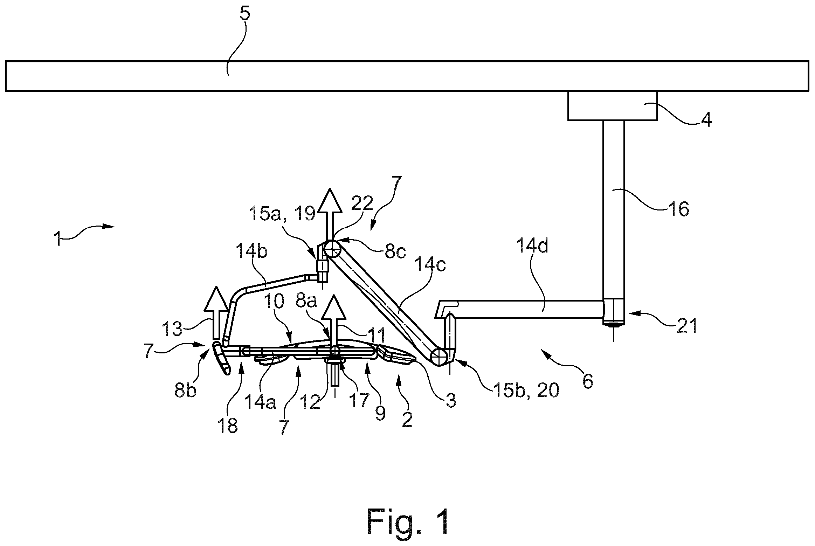

Die einzige Fig. 1 zeigt eine Seitendarstellung einer erfindungsgemäßen Operationsleuchte nach einem bevorzugten Ausführungsbeispiel, wobei verschiedene mögliche Positionen der Lagedetektionseinrichtung an dem Haltearmsystem sowie an einer Leuchteneinheit der Operationsleuchte schematisch dargestellt sind. Die Figur ist lediglich schematischer Natur und dient ausschließlich dem Verständnis der Erfindung.

In Fig. 1 ist eine Operationsleuchte 1 nach einem erfindungsgemäßen Ausführungsbeispiel veranschaulicht. Die Operationsleuchte 1 ist dazu vorbereitet, an einer Decke 5 eines Raumes, etwa eines Operationssaals, in einem Krankenhaus angebracht / befestigt zu sein. In Fig. 1 ist die Operationsleuchte 1 auch bereits an der Decke 5 des Raumes angebracht. Alternativ, in weiteren Ausführungen, ist die Operationsleuchte 1 jedoch auch zum Befestigen an einer Wand oder eines Bodens des Raumes ausgestaltet.

Die Operationsleuchte 1 weist nach Fig. 1 ein Verankerungselement 4 auf, das im Betriebszustand der Operationsleuchte 1 unmittelbar an der Decke 5 befestigt ist. An dem Verankerungselement 4 schließt ein Haltearmsystem 6 an. Das Haltearmsystem 6 ist mit einem ersten Ende / Endbereich an dem Verankerungselement 4 schwenkbar angebracht / gelagert. An einem, dem ersten Ende gegenüberliegenden, zweiten Ende / Endbereich ist an dem Haltearmsystem 6 eine Leuchteneinheit 2 der Operationsleuchte 1 wiederum schwenkbar angebracht / gelagert.

Die Leuchteneinheit 2 bildet eine Leuchte / Beleuchtungsvorrichtung / Lampe aus, die an sich wiederum mehrere einzelne Lichtquellen, nämlich LEDs, aufweist. In einer Lichtquelle ist eine einzelne LED, alternativ sind jedoch auch mehrere LEDs, vorgesehen und mit einer einzelnen (zugeordneten) Linsenoptik kombiniert. Dadurch erzeugt jede Lichtquelle in ihrem bestromten Zustand während des Betriebes der Operationsleuchte 1 einen gebündelten / parallel gerichteten Lichtstrahl. Die einzelnen Lichtquellen sind in einem Gehäuse 3 der Leuchteneinheit 2 verteilt angeordnet / aufgenommen. Die Leuchteneinheit 2, die wiederum in Fig. 1 erkennbar ist, ist hierbei im Wesentlichen als Schirmleuchte ausgeführt. Folglich weist das Gehäuse 3 eine gekrümmt verlaufende Erstreckung auf. Das Gehäuse 3 ist gesamtheitlich entlang eines Kugelsegmentes verlaufend.

Die Lichtquellen sind so in dem Gehäuse 3 angeordnet, dass sie allesamt jeweils mit ihrem Lichtauslass / ihrer Linsenoptik zu einer gemeinsamen Lichtaustrittsseite 9 des Gehäuses 3 hin gerichtet sind. Jene Lichtaustrittsseite 9 des Gehäuses 3 ist die konkave Unterseite 9 des Gehäuses 3. Somit erzeugen die Lichtquellen im Betrieb der Operationsleuchte 1 in ihrem eingeschalteten Zustand einen Lichtstrahl, der zu der gemeinsamen Lichtaustrittsseite 9 hin austritt. Die Lichtquellen der Leuchteneinheit 2 erzeugen somit ein zu einer gemeinsamen, gedachten horizontalen Beleuchtungsebene (wie einer Oberfläche eines Operationstisches) hin gerichtetes Licht.

Die der Lichtaustrittsseite 9 abgewandte Seite des Gehäuses 3 ist als Oberseite 10 bezeichnet. Diese Oberseite 10 ist eine konvexe Seite des sich schirmförmig erstreckenden Gehäuses 3.

Zur schwenkbaren sowie verschiebbaren Lagerung der Leuchteneinheit 2 an dem Verankerungselement 4 ist das kardanische Haltearmsystem 6 vorgesehen. Das Haltearmsystem 6 weist einen rohrförmigen Basisabschnitt 16 auf, der wiederum fest, nämlich vorzugsweise verdreh- sowie verschiebefest an dem Verankerungselement 4 befestigt ist. Alternativ kann der Basisabschnitt 16 jedoch auch Bestandteil des Verankerungselementes 4 sein, wobei der Basisabschnitt dann bevorzugt stoffeinteilig mit dem Verankerungselement 4 ausgeführt ist.

An dem Basisabschnitt 16 sind mehrere Tragarmabschnitte 14a bis 14d relativ zu dem Basisabschnitt 16 sowie relativ zueinander beweglich angebracht. Ein erster Tragarmabschnitt 14a ist an dem zweiten Ende des Haltearmsystems 6 ausgestaltet und an der Leuchteneinheit 2 angebracht. Die Leuchteneinheit 2 weist hierfür einen Lagerpunkt 12 auf, der eine Schwenkachse, die in Fig. 1 in die Zeichnungsebene hinein verläuft, ausbildet. Der Lagerpunkt 12 ist durch einen zapfenförmigen Vorsprung, der fest am Gehäuse 3 angebracht ist, ausgestaltet. Der Lagerpunkt 12 bildet somit ein erstes (einachsiges) Drehgelenk 17 aus.

Ein zweiter Tragarmabschnitt 14b des Haltearmsystems 6 ist wiederum schwenkbar / drehbar mit dem ersten Tragarmabschnitt 14a gekoppelt. Hierzu dient ein zweites Drehgelenk 18, das auch als Kardangelenk ausgeführt sein kann. Der zweite Tragarmabschnitt 14b ist im Wesentlichen L-förmig gebogen ausgestaltet. Der zweite Tragarmabschnitt 14b ist wiederum über ein drittes Drehgelenk 19 mit einem dritten Tragarmabschnitt 14c verbunden. Das dritte Drehgelenk 19 ist als (erstes) Kardangelenk 15a ausgestaltet. Der dritte Tragarmabschnitt 14c ist wiederum mit einem vierten Tragarmabschnitt 14d gelenkig über ein viertes Drehgelenk 20 verbunden. Auch das vierte Drehgelenk 20 ist als (zweites) Kardangelenk 15b ausgeführt. Der vierte Tragarmabschnitt ist um eine Rotationsachse (einachsig) relativ zu dem Basisabschnitt 16 mittels eines fünften Drehgelenks 21 schwenkbar gelagert. Durch die Zusammenwirkung der Tragarmabschnitte 14a bis 14d sowie der Drehgelenke 17 bis 21 ergibt sich das kardanische Haltearmsystem 6, das eine vielseitige Positionierung der Leuchteneinheit 2 im Raum ermöglicht.

Erfindungsgemäß, wie in Fig. 1 mit den drei Referenzabschnitten 8; 8a, 8b, 8c angedeutet, ist zusätzlich zumindest eine Lagedetektionseinrichtung 7 / Lageermittlungseinrichtung an der Operationsleuchte 1 vorhanden. Die Lagedetektionseinrichtung 7 ist derart beschaffen sowie angeordnet ist, dass sie im Betriebszustand einen Wert zum Ermitteln eines Abstandes zwischen einem Referenzabschnitt 8; 8a, 8b, 8c an der Leuchteneinheit 2 oder an dem Haltearmsystem 6 und der Raumdecke 5 erfasst. Die Lagedetektionseinrichtung 7 kann dabei, wie in Fig. 1 angedeutet, mehrere, nämlich drei nachfolgend näher beschriebene Sensoren 11, 13 oder 22 an den Referenzabschnitten 8a, 8b und 8c, oder alternativ nur einige / einzelne Sensoren 11, 13 und/oder 22, aufweisen. Der jeweilige Sensor 11, 13 oder 22 dient dabei stets zum Erfassen eines Wertes, der wiederum zur Ermittlung / zum Rückbezug auf die Lage / Höhe der Leuchteneinheit 2 verwendet wird.

An einem ersten Referenzabschnitt 8a der Operationsleuchte 1 ist ein (erster) Abstandserfassungssensor 11 der Lagedetektionseinrichtung 7 vorgesehen, der fähig ist, einen Abstand hin zu einem Objekt direkt zu erfassen. Der erste Abstandserfassungssensor 11 ist lediglich mittels eines Bezugspfeiles der Übersichtlichkeit halber dargestellt. Insbesondere ist der erste Abstandserfassungssensor 11 in dem Gehäuse 3 angebracht. Der erste Abstandserfassungssensor 11 ist als Infrarotsensor ausgeführt. Der erste Abstandserfassungssensor 11 hat eine Messreichweite (Länge / Erstreckung des Messfelderfassungsbereichs) von mindestens 1,50 m, bevorzugt von mindestens 2 m. Der erste Abstandserfassungssensor 11 ist zu der Oberseite 10 des Gehäuses 3 hin angebracht. Der erste Abstandserfassungssensor 11 weist einen Messfelderfassungsbereich auf, der stets, mit zumindest einem bestimmten Anteil entgegen der wirkenden Schwerkraft, d.h. räumlich in der geplanten Einbaulage nach oben ausgerichtet ist. Der erste Abstandserfassungssensor 11 erfasst daher eine Höhenverstellung der Leuchteneinheit 2 direkt, durch eine relative Abstandsmessung zwischen dem ersten Referenzabschnitt 8a / dem Gehäuse 3 und der Decke 5.

Die Leuchteneinheit 2 ist in diesem Ausführungsbeispiel nicht vollständig um 360° verschwenkbar, sondern nur in einem derartigen Bereich, bspw. um 165° entgegen sowie mit dem Uhrzeigersinn in der Ebene nach Fig. 1, dass in jeglicher Schwenkposition der Leuchteneinheit 2 eine Richtungskomponente des Messfelderfassungsbereichs des ersten Abstandserfassungssensors 11 hin zu der Decke 5 gerichtet ist. Der erste Abstandserfassungssensor 11 ist entlang der gedachten Schwenkachse an dem Gehäuse 3 angebracht.

Der erste Abstandserfassungssensor 11 ermittelt somit im Betrieb der Operationsleuchte 1 stetig einen Abstand des ersten Referenzabschnittes 8a / des Gehäuses 3 (im Bereich der Schwenkachse) relativ zu der Decke 5.

An einem zweiten Referenzabschnitt 8b, der benachbart zu dem zweiten Drehgelenk 18, nämlich an dem zweiten Tragarmabschnitt 14b, vorgesehen ist, ist ein weiterer (zweiter) Abstandserfassungssensor 13 der Lagerermittlungseinrichtung 7 angeordnet. Der zweite Abstandserfassungssensor 13 ist wie der erste Abstandserfassungssensor 11 aufgebaut sowie funktionierend. Der zweite Abstandserfassungssensor 13 ist an einer Oberseite des zweiten Tragarmabschnittes 14b, d.h. einer der Decke 5 im Betriebszustand zugewandten Seite des zweiten Tragarmabschnittes 14b, angeordnet. Der zweite Tragarmabschnitt 14b wird beim Absenken oder Anheben der Leuchteneinheit 2 entsprechend ebenfalls in der Höhe (im Raum) / in seinem Abstand relativ zur Decke 5 verschoben. Der zweite Abstandserfassungssensor 13 dient wiederum zum direkten Erfassen des Abstandes zwischen dem zweiten Referenzabschnitt 8b (an dem zweiten Tragarmabschnitt 14b) und der Decke 5. Dieser zweite Abstanderfassungssensor 13 der Lagerermittlungseinrichtung 7 kann jedoch auch prinzipiell an beliebig vielen weiteren Positionen des zweiten Tragarmabschnittes 14b bzw. der anderen Tragarmabschnitte 14a bis 14d angeordnet sein.

Die Lagedetektionseinrichtung 7 weist somit bei Verwendung des jeweiligen Abstandserfassungssensors 11 bzw. 13 einen zu einer Oberseite, d.h. zu einer nach oben hin projizierten Seite der Operationsleuchte 1, angebrachten Sensor 11, 13 auf. Mit der Richtung "nach oben" ist hierbei insbesondere eine Richtung entgegen der wirkenden Schwerkraft / Erdanziehungskraft bzw. eine vertikale Richtung in einem räumlichen Koordinatensystem gemeint.

An dem dritten Referenzabschnitt 8c, der im Bereich des dritten Drehgelenks 19 vorgesehen bzw. im dritten Drehgelenk 19 integriert ist, weist die Lagedetektionseinrichtung 7, alternativ oder zusätzlich zu dem jeweiligen Abstandserfassungssensor 11, 13, einen Winkelerfassungssensor 22 auf. Der dritte Referenzbereich 8c ist in dieser Ausführung ein Drehgelenk des Haltearmsystems 6. Der Winkelerfassungssensor 22 dient zur indirekten Ermittlung des Abstandes zwischen dem ersten Drehgelenk 19 / dem dritten Referenzbereich 8c und der Decke 5. Der Winkelerfassungssensor 22 erfasst eine (relative) Winkellage / einen Winkel zwischen einer Längsachse des sich gerade erstreckenden dritten Tragarmabschnittes 14c und einer Referenzgeraden (etwa einer gedachten Geraden, die durch das Verankerungselement 4 definiert ist oder einer Längsachse des Basisabschnittes 16), sodass indirekt ein Rückschluss auf die aktuelle Position / Lage / Höhe der Leuchteinheit 2 getroffen werden kann. Die weitere Rückbezugsberechnung des tatsächlichen Abstands zwischen der Decke 5 und dem dritten Referenzbereich 8c anhand der gemessenen Winkellage erfolgt dann vorzugsweise mit der Lagedetektionseinrichtung 7 oder alternativ in einer mit ihr verbundenen, nachfolgend näher beschriebenen Steuereinheit.

Der Winkelerfassungssensor 22 kann prinzipiell zusätzlich oder alternativ zu dem ersten und/oder zweiten Abstandserfassungssensor 11, 13 in der Operationsleuchte 1 / Lagedetektionseinrichtung 7 vorgesehen sein.

Alternativ zu dem das erste Kardangelenk 15a ausbildenden dritten Drehgelenk 19, ist es auch möglich den Winkelerfassungssensor 22 in einem anderen Bereich des Haltearmsystems 6, etwa in / an dem vierten Drehgelenk 20 vorzusehen.

Die Lagedetektionseinrichtung 7 ist auf typische Weise weiter mit einer hier der Übersichtlichkeit halber nicht weiter dargestellten Steuereinheit datenübermittelnd, nämlich elektrisch, verbunden. Die Steuereinheit ist in dem Gehäuse 3 der Leuchteneinheit 2 angebracht. Die Steuereinheit wirkt so mit den Lichtquellen steuernd zusammen, dass sie auf mehrere Lichteinstellungen / -eigenschaften / Beleuchtungseinstellungen der Leuchteneinheit 2 im Betriebszustand (in Abhängigkeit der durch die Lagedetektionseinrichtung 7 erfassten / ermittelten relativen Höhe der Leuchteneinheit 2 zur Decke 5) verstellend einwirkt. Die Lichteigenschaften, die durch die Steuereinheit einstellbar sind, sind in diesem Ausführungsbeispiel zumindest eine erzeugte Lichtfeldgröße (des durch die bestromten Lichtquellen Lichtfeldes), eine Lichtfarbe (eines erzeugten Lichtfeldes) sowie eine Anzahl der bestromten Lichtquellen. Die Steuereinheit ist so programmiert, dass sie die jeweilige Lichteigenschaft in Abhängigkeit des mittels der Lagedetektionseinrichtung 7 in dem Betriebszustand der Operationsleuchte 1 ermittelten Abstands zwischen dem Referenzabschnitt 8; 8a, 8b, 8c sowie der Raumdecke 5 einstellt.

Die Steuereinheit ist zudem vorteilhafterweise mit einer Rücksetzungseinrichtung verbunden, die es während des Betriebs der Operationsleuchte 1 ermöglicht, einen Soll-Zustand der Operationsleuchte 1 zu definieren, indem ein Ist-Zustand des Abstands relativ zu der Raumdecke 5 erfasst wird und gleichzeitig die dann aktuell eingestellten Lichteigenschaften der Leuchteneinheit 2 zusammen mit dem Abstandswert (bzw. einem Höhenwert) abgespeichert wird. Die Rücksetzungseinrichtung weist einen Rücksetzknopf und/oder eine Sprachbefehlseingabeeinrichtung, wie einen Lautsprecher, auf und ist wiederum mit der Steuereinheit elektrisch / datenübermittelnd verbunden.

In anderen Worten ausgedrückt, ist somit in einer erfindungsgemäß Operationsleuchte 1 ein Sensor 11, 13 zur Abstandsmessung der Leuchteneinheit 2 relativ zur Decke 5 angebracht. Dieser Sensor 11, 13 zur Abstandsmessung befindet sich bspw. auf der Rückseite / Oberseite 10 der Operationsleuchte 1 oder an einem der Kardangelenke 15a, 15b der Operationsleuchte 1. Ebenso könnte auch der Winkel des höhenverstellbaren Federarmes in Form des dritten Tragarmabschnittes 14c (durch einen Sensor 22) erfasst werden, um die Höhe der Operationsleuchte 1 (mittels der Steuereinheit) zu bestimmen.

### Bezugszeichenliste

- 1: Operationsleuchte
- 2: Leuchteneinheit
- 3: Gehäuse
- 4: Verankerungselement
- 5: Decke
- 6: Haltearmsystem
- 7: Lagedetektionseinrichtung
- 8: Referenzabschnitt
- 8a: erster Referenzabschnitt
- 8b: zweiter Referenzabschnitt
- 8c: dritter Referenzabschnitt
- 9: Lichtaustrittsseite / Unterseite
- 10: Oberseite
- 11: erster Abstandserfassungssensor
- 12: Lagerpunkt
- 13: zweiter Abstandserfassungssensor
- 14a: erster Tragarmabschnitt
- 14b: zweiter Tragarmabschnitt
- 14c: dritter Tragarmabschnitt
- 14d: vierter Tragarmabschnitt
- 15a: erstes Kardangelenk
- 15b: zweites Kardangelenk
- 16: Basisabschnitt
- 17: erstes Drehgelenk
- 18: zweites Drehgelenk
- 19: drittes Drehgelenk
- 20: viertes Drehgelenk
- 21: fünftes Drehgelenk
- 22: Winkelerfassungssensor

## Patentansprüche

1. Operationsleuchte (1) mit einer Leuchteneinheit (2), die eine Vielzahl einzelner in einem Gehäuse (3) aufgenommener Lichtquellen aufweist, wobei das Gehäuse (3) eine gekrümmt verlaufende Erstreckung aufweist, und wobei jede Lichtquelle so angeordnet und ausgerichtet ist, dass sie in einem bestromten Zustand einen Lichtstrahl erzeugt und die Lichtstrahlen zu einer gemeinsamen Lichtaustrittseite (9) des Gehäuses (3) hin austreten, ferner mit einem Verankerungselement (4), das zum Befestigen an einer Decke (5), einer Wand oder einem Boden eines Raumes vorbereitet ist, sowie mit einem die Leuchteneinheit (2) verschiebbar und/oder verschwenkbar an dem Verankerungselement (4) anbindenden Haltearmsystem (6), **dadurch gekennzeichnet, dass** eine einen ersten Abstandserfassungssensor (11, 13) aufweisende Lagedetektionseinrichtung (7) auf einer der Lichtaustrittsseite (9) abgewandten Oberseite (10) des Gehäuses (3) angebracht ist und derart angeordnet sowie beschaffen ist, dass sie im Betriebszustand einen Abstand eines Referenzabschnittes (8) der Leuchteneinheit (2) oder des Haltearmsystems (6) hin zu der Decke (5) ermittelt.

2. Operationsleuchte (1) nach Anspruch 1, **dadurch gekennzeichnet, dass** der erste Abstandserfassungssensor (11) im Bereich einer Schwenkachse, um die die Leuchteneinheit (2) relativ zu dem Haltearmsystem (6) schwenkbar gelagert ist, angeordnet ist.

3. Operationsleuchte (1) nach Anspruch 1 oder 2, **dadurch gekennzeichnet, dass** die Lagedetektionseinrichtung (7) einen zweiten Abstandserfassungssensor (13) aufweist, der an dem Haltearmsystem (6) vorgesehen ist.

4. Operationsleuchte (1) nach Anspruch 3, **dadurch gekennzeichnet, dass** der zweite Abstandserfassungssensor (13) an einem Kardangelenk (15a, 15b) des Haltearmsystems (6) angeordnet ist.

5. Operationsleuchte (1) nach einem der Ansprüche 1 bis 4, **dadurch gekennzeichnet, dass** die Lagedetektionseinrichtung (7) einen Winkelerfassungssensor (22) aufweist, der so beschaffen und angeordnet ist, dass er einen Winkel eines verschwenkbar zu dem Verankerungselement (4) angeordneten Tragarmabschnittes (14c) des Haltearmsystems (6) erfasst.

6. Operationsleuchte (1) nach einem der Ansprüche 1 bis 5, **dadurch gekennzeichnet, dass** die Lagedetektionseinrichtung (7) mit einer in dem Gehäuse (3) der Leuchteneinheit (2) angebrachten Steuereinheit datenübermittelnd verbunden ist.

7. Operationsleuchte (1) nach Anspruch 6, **dadurch gekennzeichnet, dass** die Steuereinheit so steuernd mit den Lichtquellen gekoppelt ist, dass zumindest eine Lichteigenschaft der Leuchteneinheit (2) in Abhängigkeit eines durch die Steuereinheit ausgegebenen Steuerbefehls einstellbar ist.

8. Operationsleuchte (1) nach Anspruch 6 und 7, **dadurch gekennzeichnet, dass** die Steuereinheit so programmiert ist, dass sie die zumindest eine Lichteigenschaft in Abhängigkeit des mittels der Lagedetektionseinrichtung (7) in einem Betriebszustand der Operationsleuchte (1) ermittelten Abstands zwischen dem Referenzabschnitt (8) und der Decke (5) einstellt.

## Claims

1. A surgical light (1) comprising a light unit (2) which has a plurality of light sources individually accommodated in a housing (3), wherein the housing (3) has a curved extension, and wherein each light source is arranged and oriented in such way that the light source produces a light beam in an energized state and the light beams are emitted toward a joint light emission side (9) of the housing (3), further having an anchoring element (4) which is prepared for fastening to a ceiling (5), a wall or a floor of a room, and a retaining arm system (6) which connects the light unit (2) slidably and/or pivotably to the anchoring element (4), **characterized in that** a position detection device (7) comprising a first distance detection sensor (11) is arranged on an upper side (10) of the housing (3) facing away from the light emission side (9) and is arranged and designed in such way that, in the operating state, the position detection device determines a distance of a reference section (8) of the light unit (2) or of the retaining arm system (6) from the ceiling (5).

2. The surgical light (1) according to claim 1, **characterized in that** the first distance detection sensor (11) is arranged in the area of a pivot axis about which the light unit (2) is pivoted relative to the retaining arm system (6).

3. The surgical light (1) according to claim 1 or 2, **characterized in that** the position detection device (7) includes a second distance detection sensor (13) provided on the retaining arm system (6).

4. The surgical light (1) according to claim 3, **characterized in that** the second distance detection sensor (13) is arranged on a universal joint (15a, 15b) of the retaining arm system (6).

5. The surgical light (1) according to one of the claims 1 to 4, **characterized in that** the position detection device (7) includes an angle detection sensor (22) which is designed and arranged such that it detects an angle of a supporting arm portion (14c) of the retaining arm system (6) arranged pivotably relative to the anchoring element (4).

6. The surgical light (1) according to any one of the claims 1 to 5, **characterized in that** the position detection device (7) is connected for data transmission to a control unit arranged in the housing (3) of the light unit (2).

7. The surgical light (1) according to claim 6, **characterized in that** the control unit is coupled to the light sources to control the latter such that at least one light characteristic of the light unit (2) can be adjusted in response to a control instruction output by the control unit.

8. The surgical light (1) according to claim 6 and 7, **characterized in that** the control unit is programmed so that it adjusts the at least one light characteristic in response to the distance between the reference section (8) and the ceiling (5) determined by means of the position detection device (7) in an operating state of the surgical light (1).

## Revendications

1. Lampe scialytique (1) avec une unité de lampe (2) qui présente une pluralité de sources de lumière individuelles reçues dans un boîtier (3), dans laquelle le boîtier (3) présente une étendue s'étendant courbée, et dans laquelle chaque source de lumière est agencée
et orientée de sorte qu'elle génère dans un état alimenté en courant un faisceau de lumière et les faisceaux de lumière sortent vers un côté de sortie de lumière (9) commun du boîtier (3), de plus avec un élément d'ancrage (4) qui
est préparé pour la fixation à un plafond (5), une paroi ou un sol d'une pièce, ainsi qu'avec un système de bras de retenue (6) reliant l'unité de lampe (2) de manière mobile et/ou pivotante à l'élément d'ancrage (4), **caractérisée en ce qu'**un dispositif de détection de position (7) présentant un premier capteur de détection de distance (11, 13) est monté sur un côté supérieur (10) éloigné du côté de sortie de lumière (9) du boîtier (3) et est agencé ainsi que conçu de telle manière qu'il détermine dans l'état de fonctionnement une distance d'une section de référence (8) de l'unité de lampe (2) ou du système de bras de retenue (6) vers le plafond (5).

2. Lampe scialytique (1) selon la revendication 1, **caractérisée en ce que** le premier capteur de détection de distance (11) est agencé dans la zone d'un axe de pivotement, autour duquel l'unité de lampe (2) est logée de manière pivotante par rapport au système de bras de retenue (6).

3. Lampe scialytique (1) selon la revendication 1 ou 2, **caractérisée en ce que** le dispositif de détection de position (7) présente un second capteur de détection de distance (13) qui est prévu sur le système de bras de retenue (6).

4. Lampe scialytique (1) selon la revendication 3, **caractérisée en ce que** le second capteur de détection de distance (13) est agencé au niveau d'une articulation de Cardan (15a, 15b) du système de bras de retenue (6).

5. Lampe scialytique (1) selon l'une quelconque des revendications 1 à 4, **caractérisée en ce que** le dispositif de détection de position (7) présente un capteur de détection angulaire (22) qui est conçu et agencé de sorte
qu'il détecte un angle d'une section de bras porteur (14c) agencé de manière pivotante par rapport à l'élément d'ancrage (4) du système de bras de retenue (6).

6. Lampe scialytique (1) selon l'une quelconque des revendications 1 à 5, **caractérisée en ce que** le dispositif de détection de position (7) est relié à un dispositif de commande monté dans le boîtier (3) de l'unité de lampe (2) de manière à transmettre des données.

7. Lampe scialytique (1) selon la revendication 6, **caractérisée en ce que** le dispositif de commande est couplé de manière à commander aux sources de lumière, de sorte qu'au moins une propriété de lumière de l'unité de lampe (2) soit réglable en fonction d'un ordre de commande sorti par le dispositif de commande.

8. Lampe scialytique (1) selon les revendications 6 et 7, **caractérisée en ce que** le dispositif de commande est programmé de sorte qu'il règle l'au moins une propriété de lumière en fonction de la distance déterminée au moyen du dispositif de détection de position (7) dans un état de fonctionnement de la lampe scialytique (1) entre la section de référence (8) et le plafond (5).
